(19) Europäisches Patentamt
European Patent Office
Office européen des brevets

(11) Publication number: 0 349 928
A2

(12) # EUROPEAN PATENT APPLICATION

(21) Application number: 89112002.4

(22) Date of filing: 30.06.89

(51) Int. Cl.4: C07H 19/16 , A61K 31/70

(30) Priority: 08.07.88 GB 8816345

(43) Date of publication of application:
10.01.90 Bulletin 90/02

(84) Designated Contracting States:
AT BE CH DE FR GB IT LI LU NL SE

(71) Applicant: F. HOFFMANN-LA ROCHE AG
Postfach 3255
CH-4002 Basel(CH)

(72) Inventor: Martin, Joseph Armstrong
10 The Chowns
West Common Harpenden(GB)
Inventor: Parkes, Kevin Edward Burdon
60 Hallmead
Letchworth Hertfordshire(GB)

(74) Representative: Lederer, Franz, Dr. et al
Patentanwalt Dr. Franz Lederer
Lucile-Grahn-Strasse 22
D-8000 München 80(DE)

(54) **Purine derivatives.**

(57) Purinderivate der allgemeinen Formel

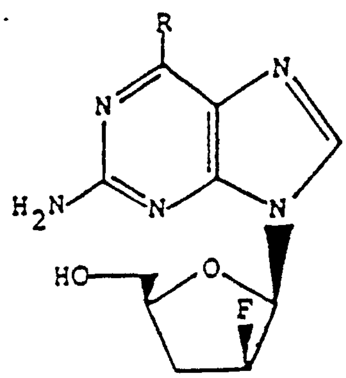

I

worin R Chlor, nieder-Alkoxy, nieder-Alkylthio oder Mono(nieder-alkyl)amino ist, haben antivirale Wirksamkeit und können für die Behandlung oder Prophylaxe von humanen oder veterinären viralen Infektionen insbesondere von retroviralen Infektionen wie HIV verwendet werden. Man kann sie aus 2-Amino-9-(5-O-aroyl-2,3-dideoxy-2-fluor-β-D -threopentofuranosyl)-6-chlor-9H-purinen herstellen.

EP 0 349 928 A2

## Purinderivate

Die vorliegende Erfindung betrifft Purinderivate der allgemeinen Formel

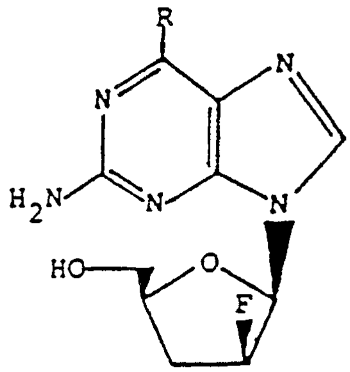

I

worin R Chlor, nieder-Alkoxy, nieder Alkylthio oder Mono(nieder-alkyl)amino ist.

Diese Verbindungen sind neu und haben wertvolle pharmakodynamische Eigenschaften. Insbesondere haben sie antivirale Wirksamkeit und können in der Behandlung und Prophylaxe von viralen Infektionen beim Menschen und beim Tier im speziellen von retroviralen Infektionen wie HIV und dergleichen Verwendung finden.

Gegenstand der vorliegenden Erfindung sind die weiter oben definierten Verbindungen als solche, sowie zur Verwendung als therapeutisch aktive Substanzen, ein Verfahren zur Herstellung dieser Verbindungen, Zwischenprodukte für die Herstellung dieser Verbindungen, Medikamente auf der Basis dieser Verbindungen und die Verwendung dieser Verbindungen zur Behandlung und Prophylaxe von Krankheiten insbesondere von viralen Infektionen, oder bei der Herstellung von Medikamenten für die Behandlung oder Prophylaxe von viralen Infektionen.

Im Rahmen der Erfindung bedeutet "nieder-Alkyl" allein oder in Verbindung, z.B. in "nieder-Alkoxy", eine geradkettige oder verzweigte Alkylgruppe mit 1-6 vorzugsweise 1-4 C-Atome, wie Methyl, Aethyl n-Propyl, Isopropyl, n-Butyl, s-Butyl, t-Butyl und n-Pentyl. Beispiele von "nieder-Alkoxygruppen" sind Methoxy, Aethoxy, n-Propoxy, Isopropoxy, n-Butoxy, s-Butoxy, t-Butoxy und n-Pentyloxy. Beispiele von "nieder-Alkylthiogruppen" sind Methylthio, Aethylthio und n-Propylthio.

Die obigen Verbindungen können in Form von Tautomeren vorliegen, die ebenfalls Gegenstand der Erfindung sind.

Bevorzugte Verbindungen der Formel I sind die folgenden:
2-Amino-6 chlor-9-(2,3-dideoxy-2-fluor-β-D-threopentofuranosyl)-9H-purin,
2-Amino-9-(2,3-dideoxy-2-fluor-β-D-threopentofuranosyl)-6-methoxy-9H-purin,
2-Amino-9-(2,3 dideoxy-2-fluor-β-D-threopentofuranosyl)-6-methylthio-9H-purin und
2-Amino-9-(2,3-dideoxy-2-fluor-β-D-threopentofuran osyl)-6-methylamino-9H-purin.

Die obigen Verbindungen können erfindungsgemäss dadurch hergestellt werden, dass man a) zur Herstellung einer Verbindung der Formel I, in der R Chlor ist, die Gruppe $R^1$ aus einer Verbindung der allgemeinen Formel

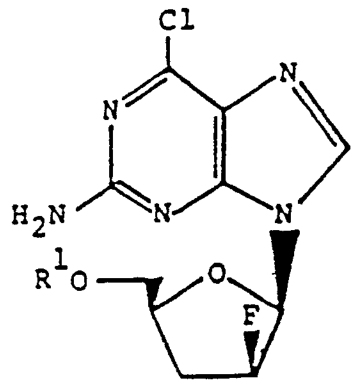

II

worin $R^1$ Aroyl, vorzugsweise Benzoyl ist, abspaltet, oder

b) zur Herstellung einer Verbindung der Formel I, in der R nieder Alkoxy ist, eine Verbindung der Formel II mit einem Alkalimetall-nieder-alkoxid umsetzt, oder

c) zur Herstellung einer Verbindung der Formel I, in der R nieder-Alkylthio ist, eine Verbindung der Formel II mit einem Alkalimetall-nieder-alkanthiolat umsetzt und die Gruppe $R^1$ aus dem erhaltenen Produkt abspaltet, oder

d) zur Herstellung einer Verbindung der Formel I, in der R Mono(nieder alkyl)amino ist, eine Verbindung der Formel II mit einem nieder-Alkylamin umsetzt.

Die Abspaltung einer Gruppe $R^1$ aus einer Verbindung der Formel II nach der Verfahrensvariante a) kann durch Behandlung mit Ammoniak bei Raumtemperatur durchgeführt werden. Zweckmässig wird eine Lösung von Ammoniak in einem nieder-Alkanol wie Methanol verwendet.

Die Reaktion einer Verbindung der Formel II mit einem Alkalimetall-nieder-alkoxid, z.B. Natriummethoxid, oder einem Alkalimetall-nieder-alkanthiolat, z.B. Natriummethan thiolat, nach der Verfahrensvariante b) oder c) kann in einem inerten organischen Lösungsmittel wie einem nieder-Alkanol z.B. Methanol und bei erhöhter Temperatur zweckmässig unter Rückfluss durchgeführt werden.

Die Abspaltung einer Gruppe $R^1$ aus dem Produkt der Reaktion einer Verbindung der Formel II mit einem Alkalimetall-nieder-alkanthiolat kann wie für die Verfahrensvariante a) beschrieben durchgeführt werden.

Die Reaktion einer Verbindung der Formel II mit einem nieder-Alkylamin, z.B. Methylamin, nach der Verfahrensvariante d) kann mittels einer Lösung eines nieder-Alkylamins in einem nieder-Alkanol, z.B. Aethanol, bei erhöhter Temperatur in einem Bombenrohr durchgeführt werden.

Die Verbindungen der Formel II sind neu und als solche Gegenstand der Erfindung. Sie können z.B. durch Reaktion des 2-Amino-6-chlorpurins mit einer Verbindung der Formel

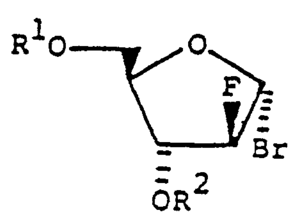

III

worin $R^1$ die obige Bedeutung hat und $R^2$ Aroyl, vorzugsweise Benzoyl ist,
Abspaltung der Gruppe $R^2$ aus der erhaltenen Verbindung der allgemeinen Formel

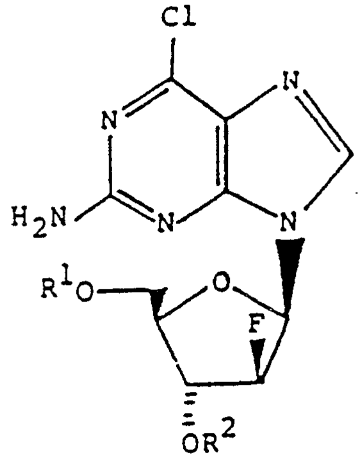

IV

Reaktion der entstandenen Verbindung der allgemeinen Formel

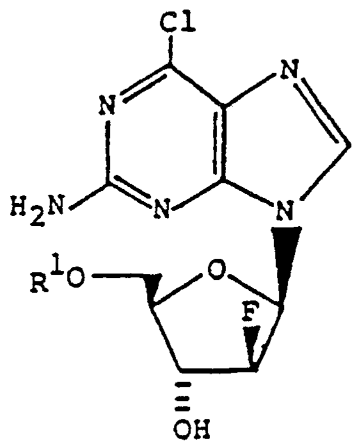

V

mit Phenoxythionocarbonylchlorid und Behandlung der entstandenen Verbindung der allgemeinen Formel

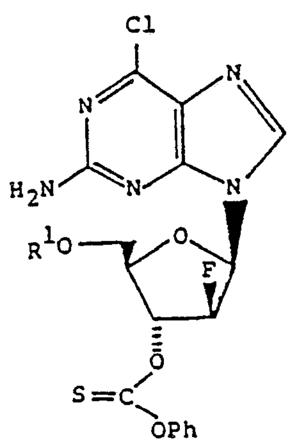

VI

worin $R^1$ die obige Bedeutung hat und Ph Phenyl ist,
mit Tributylzinnhydrid in Gegenwart eines freie Radikale erzeugenden Mittels in an sich bekannter Weise hergestellt werden.

So wird zweckmässig das 2-Amino-6-chlorpurin, z.B. unter Verwendung von Hexamethyldisilazan vor der Reaktion mit einer Verbindung der Formel III zu einem Silylderivat aktiviert. Die Reaktion wird zweckmässig bei erhöhter Temperatur, z.B. bei Rückflusstemperatur des Reaktionsgemisches und unter einer inerten Atmosphäre z.B. Argon, durchgeführt. Dann wird die Silylgruppe, z.B. mittels einer anorganischen Säure wie Salzsäure, abgespaltet.

In der zweiten Stufe wird die Abspaltung der Gruppe $R^2$ aus einer Verbindung der Formel IV zweckmässig mittels Ammoniak, vorzugsweise einer Lösung von Ammoniak in einem Tetrahydrofuran (THF) enthaltenden nieder Alkanol, z.B. Methanol, bei Raumtemperatur bewerkstelligt.

In der nächsten Stufe wird die Reaktion einer Verbindung der Formel V mit Phenoxythionocarbonylchlorid zweckmässig in einem inerten organischen Lösungsmittel, wie Acetonitril und in Gegenwart eines Säure-bindenden Mittels wie einem tertiären Amin, z.B. Pyridin oder 4-Dimethylaminopyridin durchgeführt. Diese Reaktion wird vorzugsweise bei Raumtemperatur und unter einer inerten Atmosphäre wie Argon bewerkstelligt.

In der letzten Stufe wird die Behandlung einer Verbindung der Formel VI mit Tributylzinnhydrid in Gegenwart eines freie Radikale erzeugenden Mittels, z.B. Azobisisobutyronitril, zweckmässig in einem inerten organischen Lösungsmittel wie einem aromatischen Kohlenwasserstoff, z.B. Benzol oder Toluol, bei erhöhter Temperatur, z.B. etwa 60-90 °C und unter einer inerten Atmosphäre, wie Argon, durchgeführt.

Das 2-Amino 6-chlorpurin sowie die Verbindung der Formel III, in der $R^1$ und $R^2$ Benzoyl sind, sind bekannte Verbindungen. Die anderen Verbindungen der Formel III können in Analogie zu den bekannten Verbindungen hergestellt werden.

Die Aktivität der Purinderivate der vorliegenden Erfindung gegen HIV kann wie folgt nachgewiesen werden.

In diesem Versuch verwendet man HTLV III, gezüchtet in C8166-Zellen (eine menschliche $CD4^+$ T-Lymphoblastenlinie), RPM1 1640 Medium mit Bicarbonatpuffer, Antibiotika und 10%igem fötalem Rinderserum. Eine Zellsuspension wird mit der 10-fachen $TCD_{50}$ des Virus infiziert. Man lässt 90 Minuten bei 37 °C adsorbieren. Die Zellen werden mit dem Medium gewaschen. Der Versuch wird in 6 ml Gewebekulturröhrchen durchgeführt. Jedes Röhrchen enthält 2 x $10^5$ infizierte Zellen in 1,5 ml Medium. Es werden 15 µl einer Lösung der Substanz in wässrigem Medium oder in DMSO zugesetzt. Nach 72 Stunden Inkubation bei 37 °C in einer feuchten 5% $CO_2$ enthaltenden Atmosphäre werden die Kulturen zentrifugiert. Ein aliquoter Teil des Ueberstandes wird solubilisiert und einem Antigenabfangversuch unterworfen unter Verwendung eines primären Antiserums mit besonderer Reaktivität gegen virales Protein 24 und eines Meerrettich-Peroxidase-Detektionssystems. Die Farberzeugung wird spektrophotometrisch gemessen und in Abhängigkeit der Konzentration der Testsubstanz dargestellt. Die einen 50%igen Schutz erzeugende Konzentration ($IC_{50}$) wird ermittelt. Für jedes der vier Produkte der Beispiele 1-4 wurde eine $IC_{50}$ von 10 µM ermittelt.

Die Puridinderivate der vorliegenden Erfindung können in Form pharmazeutischer Präparate, die diese Verbindungen zusammen mit einem verträglichen pharmazeutischen Träger enthalten, als Medikamente verwendet werden. Diese Präparate können oral, z.B. in Form von Tabletten Dragées, Hartgelatinekapseln, Weichkapseln, Lösungen, Emulsionen oder Suspensionen, oder parenteral, z.B. in Form von Injektionslösungen, verabreicht werden.

Die weiter oben erwähnten Träger können pharmazeutisch inerte, anorganische oder organische Materialien sein. Beispiele von Träger für Tabletten, Dragées und Hartkapseln sind Lactose, Maisstärke oder Derivate davon, Talk, Stearinsäure oder Salze davon. Beispiele von Träger für Weichkapseln sind pflanzliche Oele, Wachse, Fette, halbfeste und flüssige Polyole. Beispiele von Träger für die Herstellung von Lösungen oder Sirups sind Wasser, Polyole, Saccharose, Invertzucker und Glucose. Beispiele von Träger für Injektionslösungen sind Wasser, Alkohole, Polyole, Glycerin und pflanzliche Oele.

Die pharmazeutischen Präparate können auch herkömmliche pharmazeutische Hilfsmittel enthalten, wie Konservierungs-, Solubilisierungs-, Stabilisierungs-, Befeuchtungsmittel, Emulgatoren, Süssmittel, Farb- oder Riechstoffe, Salze zur Veränderung des osmotischen Druckes, Puffer, Ueberzugsmittel oder Antioxidantien. Sie können auch andere therapeutisch wertvolle Substanzen enthalten.

Die Dosierung, in der die Purinderivate der vorliegenden Erfindung verabreicht werden können, kann nach oben und unten in Abhängigkeit von Faktoren, wie der einzelnen zu verab- reichenden Verbindung, der Schwere der zu behandelnden Erkrankung und dem Zustand des Patienten, variiert werden. Die Purinderivate der vorliegenden Erfindung kann man in einem täglichen Dosierungsbereich von etwa 0,1 bis 20, vorzugsweise 0,2 bis 15, insbesondere etwa 0,4 bis 10 mg/kg Körpergewicht verabreichen.

Diese Purinderivate können in Einzeldosen oder vorzugsweise in mehreren Dosen, z.B. bis zu 6 über den Tag verabreicht werden. Eine Einzeldosis für eine solche Verabreichungsform kann z.B. von etwa 0,5 bis 300, vorzugsweise etwa 1.0 bis 300 und insbesondere etwa 2.0 bis 200 mg Purinderivat enthalten.

Die pharmazeutischen Präparate kann man so herstellen, dass man die obigen Purinderivate, gewünschenfalls zusammen mit anderen therapeutisch wertvollen Substanzen, mit einem verträglichen pharmazeutischen Träger und gewünschtenfalls einem pharmazeutischen Hilfsstoff vermischt und das Gemisch in die gewünschte Verabreichungsform bringt.

Beispiel 1

100 mg 2-Amino-9-(5-O-benzoyl 2,3-dideoxy 2-fluor-β-Dthreopentofuranosyl)-6-chlor-9H-purin werden in 5 ml mit Ammoniak gesättigtem Methanol gelöst und das Gemisch wird 22 Stunden bei Raumtemperatur stehengelassen. Die Lösung wird unter vermindertem Druck zur Trockene eingedampft und der Rückstand wird zwischen Wasser und Cyclohexan verteilt. Die wässrige Phase wird filtriert und das Filtrat wird gefriergetrocknet. Man erhält 55 mg 2 Amino-6-chlor-9-(2,3-dideoxy-2-fluor-β-D -threopentofuranosyl)-9H-purin. Umkristallisation aus Wasser ergibt 28 mg reines Produkt vom Schmelzpunkt 95-100 °C.

Das Ausgangsmaterial wird wie folgt hergestellt:

A) Ein Gemisch von 7,3 g 2-Amino-6-chlorpurin, 30 ml Hexamethyldisilazan und 10 mg Ammoniumsulfat wird 3 Stunden unter Rückfluss unter Argon erhitzt. Weitere 5 ml Hexamethyldisilazan und 10 mg Ammoniumsulfat werden zugesetzt. Nach 1 Stunde Erhitzen wird die entstandene Lösung abgekühlt, unter vermindertem Druck eingedampft und der Rückstand wird zweimal in 25 ml Toluol gelöst und jedes Mal eingedampft. Der Rückstand wird in 100 ml 1,2-Dichloräthan gelöst und einer Lösung von 18,2 g 2-Deoxy-2-fluor-3,5-di-O-benzoyl-α-D-arabinofuranosylbromid in 100 ml 1,2-Dichloräthan zugesetzt. Das Gemisch wird 6 Tage unter Rückfluss unter Argon erhitzt, dann auf Raumtemperatur abkühlen gelassen und 1 Stunde mit 100 ml einer gesättigten wässrigen Natriumbicarbonatlösung gerührt. Das Gemisch wird filtriert, die Phasen werden getrennt und die wässrige Phase wird mit Dichlormethan extrahiert. Eine 0,31M Lösung von Salzsäure in Chloroform wird der organischen Phase zugesetzt und nach 30 Minuten wird das Gemisch mit gesättigter wässriger Natriumbicarbonatlösung gewaschen, getrocknet und unter vermindertem Druck eingedampft. Der Rückstand wird durch Flashchromatographie über Silicagel mit Cyclohexan/Aethylacetat gereinigt. Man erhält 10,8 g 2-Amino-6-chlor-9-(2-deoxy-3,5-di-O-benzoyl-2-fluor -β-D-arabinofuranosyl)-9H-purin.

B) Einer Lösung von 2,0 g des Produkts von A) in 10 ml THF werden 10 ml und 2 Stunden später zusätzliche 10 ml mit Ammoniak gesättigtes Methanol zugesetzt. Nach 1 Stunde wird das Gemisch unter vermindertem Druck zur Trockene eingedampft und das entstandene Oel wird durch Flashchromatographie über Silicagel mit 10% Methanol in Dichlormethan gereinigt. Man erhält 1,23 g 2-Amino-9-(5-O-benzoyl-2-deoxy-2-fluor -β-D-arabinofuranosyl)-6-chlor-9H-purin.

C) Eine Lösung von 300 ml des Produkts von B), 210 mg 4-(Dimethylamino)pyridin und 130 µl Phenoxythionocarbonylchlorid in 9 ml Acetonitril wird 2,5 Stunden unter Argon gerührt. Die Lösung wird unter vermindertem Druck eingedampft und der Rückstand wird durch Flashchromatographie über Silicagel mit 5% Methanol in Dichlormethan gereinigt. Man erhält 0,25 g 2-Amino-9-(5-O-benzoyl-2-deoxy-2-fluor-3-O-phenoxythiocarbonyl-β-D-arabinofuranosyl)-6-chlor-9H-purin.

D) Eine Lösung von 250 mg des Produkts von C), 141 µl Tributylzinnhydrid und 15 mg Azobisisobutyronitril in 8 ml Toluol wird zur Befreiung von Sauerstoff 20 Minuten mit Argon begast. Das Gemisch wird dann 3 Stunden unter Argon bei 75 °C erhitzt, dann auf Raumtemperatur abkühlen gelassen und unter vermindertem Druck zur Trockene eingedampft. Der Rückstand wird durch Flashchromatographie über Silicagel mit Dichlormethan/Aceton (4:1) gereinigt. Man erhält nach Abdampfen der das Produkt enthaltenden Fraktionen 140 mg 2-Amino-9-(5-O-benzoyl-2,3-dideoxy-2-fluor-β-D-threopentofuranosyl)-6-chlor-9H-purin, Smp. 194-196 °C.

Beispiel 2

0,51 ml einer 1M Lösung von Natriummethoxid in Methanol werden einer Lösung von 100 mg 2-Amino-9 (5-O-benzoyl-2,3-dideoxy-2-fluor-β-D -threopentofuranosyl)-6-chlor-9H-purin in 8,5 ml Methanol zugesetzt und das Gemisch wird 1,5 Stunden unter Rückfluss erhitzt. Die Lösung wird auf Raumtemperatur abkühlen gelassen und unter vermindertem Druck zur Trockene eingedampft. Der Rückstand wird in 5 ml Wasser gelöst und die Lösung durch tropfenweise Zugabe von 1M Salzsäure neutral gestellt. Die Lösung wird mit Methylcyclohexan gewaschen, die Extrakte werden mit Wasser gewaschen und die wässrige Phase wird filtriert und gefriergetrocknet. Der entstandene Feststoff wird aus Wasser umkristallisiert. Man erhält 34 mg 2-Amino-9-(2,3-dideoxy-2-fluor-β-D-threopentofuranosyl)-6-methoxy 9H-purin, Smp. 94-96 °C.

Beispiel 3

Eine Lösung von 200 mg 2-Amino-9-(5-O-benzoyl-2,3-dideoxy-2-fluor-β-D-threopentofuranosyl)-6-chlor-9H-purin und 80 mg Natriummethanthiolat in 10 ml Methanol wird 3 Stunden unter Rückfluss unter Argon erhitzt. Das Gemisch wird auf Raumtemperatur abkühlen gelassen und unter vermindertem Druck zur Trockene eingedampft. Der Rückstand wird in 15 ml Wasser gelöst und die Lösung durch tropfenweise Zugabe von Eisessig neutral gestellt. Die Lösung wird mit Methylcyclohexan gewaschen und unter vermindertem Druck zur Trockene eingedampft. Der Rückstand wird in Methanol gelöst und die Lösung wird eingedampft. Das entstandene Gummi wird in einer gesättigten Lösung von Ammoniak in Methanol gelöst und über Nacht bei Raumtemperatur stehengelassen. Die Lösung wird dann unter vermindertem Druck zur Trockene eingedampft und der Rückstand wird durch Flashchromatographie über Silicagel mit 10% Aceton in Aethylacetat gereinigt. Man erhält 68 mg 2-Amino-9-(2,3-dideoxy-2-fluor-β-D - threopentofuranosyl)-6-methylthio-9H-purin, Smp. 164-165 °C.

## Beispiel 4

Ein Gemisch von 120 mg 2-Amino-9-(5-O-benzoyl-2,3-dideoxy-2-fluor-β-D-threopentofuranosyl)-6-chlor-9H-purin und 20 ml einer 33%igen äthanolischen Lösung von Methylamin wird 10 Stunden im Bombenrohr bei 100 °C erhitzt. Nach Abkühlen auf Raumtemperatur wird das Gemisch unter verminder tem Druck zur Trockene eingedampft und der Rückstand wird durch Flashchromatographie über Silicagel mit Aceton/Aethylacetat (2:1) gereinigt. Man erhält 28 mg 2-Amino 9-(2,3-dideoxy-2-fluor-β-D - threopentofuranosyl)-6-methylamino-9H-purin, Smp. 96-98 °C.

## Beispiel A

Tabletten und Kapseln mit folgenden Inhaltsstoffen können in an sich bekannter Weise hergestellt werden:

| Inhaltsstoffe | | Pro Tablette |
|---|---|---|
| Wirkstoff | | 10 mg |
| Lactose | | 20 mg |
| Stärke | | 4 mg |
| Polyvinylpyrrolidon | | 0,5 mg |
| Magnesiumstearat | | 0,5 mg |
| | Tablettengewicht | 35 mg |
| Inhaltsstoffe | | Pro Kapsel |
| Wirkstoff | | 25 mg |
| Lactose | | 15 mg |
| Natriumstärkeglycollat | | 2,5 mg |
| Magnesiumstearat | | 0,5 mg |
| | Kapselfüllstoff | 43 mg |

## Claims

1. Purinderivate der allgemeinen Formel

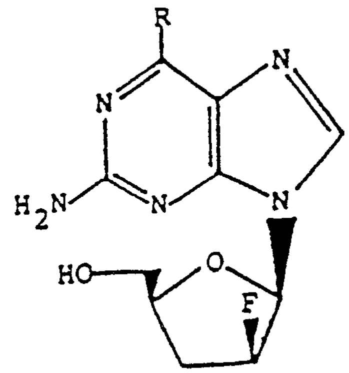

I

worin R Chlor nieder-Alkoxy, nieder-Alkylthio oder Mono(nieder-alkyl)amino ist.

2. Verbindungen nach Anspruch 1 aus der Gruppe der folgenden:
2-Amino-6-chlor-9-(2,3-dideoxy-2-fluor-β-D-threopentofuranosyl)-9H-purin,
2-Amino-9-(2,3-dideoxy-2-fluor-β-D-threopentofuranosyl)-6-methoxy-9H-purin,
2-Amino-9-(2,3-dideoxy-2-fluor-β-D-threopentofuranosyl)-6-methylthio-9H-purin und
2-Amino-9-(2,3-dideoxy-2-fluor-β-D-threopentofuranosyl)-6-methylamino-9H-purin.

3. Verbindungen der Formel

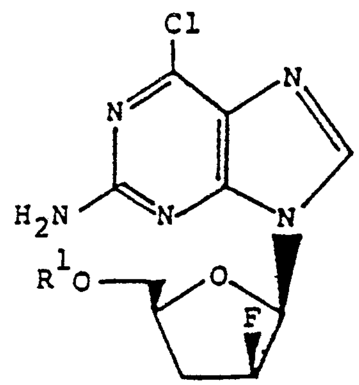

II

worin $R^1$ Aroyl ist.

4. 2-Amino-9-(5-O-benzoyl-2,3-dideoxy-2-fluor-β-D-threopentofuranosyl)-6-chlor-9H-purin.

5. Purinderivate nach Anspruch 1 oder 2 zur Anwendung als therapeutisch aktive Substanzen, insbesondere für die Behandlung oder Prophylaxe von viralen Infektionen, im speziellen von retroviralen Infektionen und insbesondere von HIV-Infektionen.

6. Verfahren zur Herstellung von Purinderivaten nach Anspruch 1 oder 2, dadurch gekennzeichnet, dass man

a) zur Herstellung einer Verbindung der Formel I, in der R Chlor ist die Gruppe $R^1$ aus einer Verbindung der Formel II gemäss Anspruch 3 abspaltet, oder

b) zur Herstellung einer Verbindung der Formel I, in der R nieder-Alkoxy ist, eine Verbindung der Formel II mit einem Alkalimetall-nieder-alkoxid umsetzt, oder

c) zur Herstellung einer Verbindung der Formel I, in der R nieder Alkylthio ist, eine Verbindung der Formel II mit einem Alkalimetall-nieder alkanthiolat umsetzt und die Gruppe $R^1$ aus dem erhaltenen Produkt abspaltet, oder

d) zur Herstellung einer Verbindung der Formel I, in der R Mono(nieder-alkyl)amino ist, eine Verbindung der Formel II mit einem nieder-Alkylamin umsetzt.

7. Ein Medikament, insbesondere zur Behandlung oder Prophylaxe von viralen Infektionen im speziellen von retroviralen Infektionen und insbesondere von HIV-Infektionen auf der Basis von einem Purinderivat nach Anspruch 1 oder 2.

8. Verwendung eines Purinderivats nach Anspruch 1 oder 2, bei der Herstellung von Medikamenten für die Behandlung oder Prophylaxe von viralen Infektionen insbesondere von retroviralen Infektionen, im speziellen von HIV-Infektionen.